# EUROPEAN PATENT APPLICATION

(11) **EP 4 607 173 A1**
(43) Date of publication of application: **27.08.2025**
(21) Application number: 24755929.7
(22) Date of filing: 25.01.2024
(51) Int. Cl.: G01N 1/28, G01N 33/48, G01N 1/36

(54) **METHOD FOR PROCESSING IONIC LIQUID PREPARATION AND TISSUE, AND APPLICATION THEREOF**

(30) Priority: 17.02.2023 CN 202310128830
(71) Applicant: Tsinghua University, Beijing 100084 (CN)
(72) Inventor: YUAN, Kexin, Beijing 100084 (CN); WANG, Tao, Beijing 100084 (CN); GAO, Yixiao, Beijing 100084 (CN); HU, Ying, Beijing 100084 (CN)
(74) Representative: Murgitroyd & Company
(86) International application number: PCT/CN2024/073964
(87) International publication number: WO 2024/169554

(57) **Abstract**

A hydrophilic amorphous ionic liquid preparation and a preparation method therefor, a biological tissue processing method using the ionic liquid preparation, and an application of the ionic liquid preparation in biological tissue processing. The ionic liquid preparation comprises: a) an ionic liquid containing a specific cation and a specific anion; b) an auxiliary agent for adjusting the properties of the components of a); and c) water.

## Description

### Technical Field

The present invention relates to an ionic liquid and use thereof, in particular, to a hydrophilic, high refractive index amorphous ionic liquid formulation, a biological tissue treatment method using the ionic liquid formulation and use of the ionic liquid in the biological tissue treatment method.

### Background

Ionic liquid is a molten salt system composed of an organic cation and an inorganic (or organic) anion, which is liquid at room temperature. Ionic liquid is odorless, non-flammable, has extremely low vapor pressure, is almost non-volatile, and has high thermal and chemical stability. Through the modification of functionalized groups, the functionalized ionic liquid that meets various requirements can be created. Based on these characteristics of the ionic liquid, it is possible to find ionic liquid formulations suitable for biological tissue treatment.

Biological tissue treatment methods include, but are not limited to, methods such as a non-deformation tissue clearing method, non-destructive cryopreservation method of tissue by vitrification at ultra-low temperature, a low-temperature fluorescence enhancement method, an ice-free cryosectioning method, a cryosectioning method for an expanded tissue, and a super-resolution imaging method.

### 1. Tissue clearing

Tissue clearing uses delipidating and decolorization processes to remove substances and pigments with uneven refractive index, and uses refractive index matching solution to match the refractive index of tissue to a higher uniform level, thereby reducing light scattering, refraction and reflection, and making the treated tissue show a transparent effect. At present, traditional refractive index matching reagents are divided into organic solvents and hydrophilic reagents.

The organic solvent clearing method includes BABB and DISCO series, which uses the substances such as alcohols or ethers (such as tetrahydrofuran) for dehydration and delipidating to remove water component with lower refractive index, and uses organic solvents for clearing. Although the organic solvents have higher refractive index and good clearing effect, their preservation effect on fluorescence is poor, and a large amount of autofluorescence may be produced, and the organic solvents with high concentration will cause great distortion to the tissues. **In** addition, reagents with relatively strong toxic such as tetrahydrofuran are contained in the organic solvent formula, which may be harmful to human health. And the dehydration process can shrink the tissue.

The aqueous clearing method can cause the tissue to present expansion phenomenon. Hydrophilic reagents are mostly based on the hyperhydration properties of urea, which will lead to the denaturation of the protein and cause the tissue to expand. Moreover, the clearing speed of many methods is slow, and the refractive index matching substances such as antipyrine are easy to crystallize and destroy the tissues when they reach saturation. **In** addition, the hydrophilic reagents have high water content, and its refractive index is difficult to reach a higher level. Due to the high water content, the transparency of the hydrophilic reagents will be lower, and in order to prevent the refractive index matching agents with high concentration from crystallizing, the cleared tissues can only be stored at room temperature, which is not friendly to fluorescence. At the same time, the rigidity of the tissue of the aqueous clearing treatment decreases, and the tissue is easy to deform and break, which is therefore not friendly to sample storage.

### 2. Cryopreservation of tissue by vitrification at ultra-low temperature

Cryopreservation of tissue by vitrification at ultra-low temperature uses cryoprotectant with high concentration and liquid nitrogen to cool down, so that dehydrated samples can be vitrified without the production of ice, thereby realizing long-term cryopreservation of the cells, the tissues or the organs, and maintaining their basic structure or viability.

**In** the glassy state, water molecules do not rearrange, do not produce structural and volume changes, and thus do not cause tissue damage due to mechanical damage or solution effects. However, a concentration of a cryoprotectant used by the existing cryopreservation technology of tissue by vitrification at ultra-low temperature is very high, and some components such as dimethyl sulfoxide (DMSO) are extremely toxic to the materials, so it is necessary to strictly control the dehydration process and the permeability of the cryoprotectant. In addition, in actual operation, cryoprotectants can only reduce, but cannot completely inhibit the formation of ice crystals, and thus still cause tissue damage to a certain extent. In addition, the existing ultra-low temperature vitrification method mainly uses liquid nitrogen quick freezing to prevent the formation of ice crystals, but the samples preserved by ultra-low temperature vitrification are easy to form recrystallized ice crystals and damage samples during the rewarming process, which makes the method be difficult to operate and be very high risky.

### 3. Low-temperature fluorescence enhancement

The fluorescence intensity has a sensitive response to temperature. When the temperature elevates, the molecule is excited to accept additional thermal energy, which may convert the excitation energy into ground state vibration energy, and then quickly vibrate and relax and lose the vibration energy. In addition, in the solution system, the decrease of temperature increases the viscosity of the medium, the collision between fluorescent substances and solvent molecules decreases, and the deactivation probability decreases. Therefore, the fluorescence intensity of the fluorescent substance at low temperature is significantly enhanced as compared with that at room temperature.

In recent years, the low-temperature properties of fluorescence have attracted more and more attention. In fluorescence imaging, photobleaching of the fluorescent proteins will be reduced at lower temperature. However, since aqueous systems are easy to form ice crystals at low temperature, causing damage to the tissues, sample preparation has become a major challenge for low-temperature fluorescence imaging. In addition, at present in the art, there is no special imaging system for fluorescence enhancement through low temperature, and most of the samples for freeze imaging are used for photoelectric combined imaging of cryogenic electron microscopy. For the samples of cryogenic electron microscope, they also face the problem of the recrystallization, and the laser that excites fluorescence may also cause the freezing and thawing of the samples, which makes this method not be popularized too much.

### 4. Cryosectioning

The cryosectioning method is a method in which the tissues reach the appropriate hardness in a shorter time in a low temperature environment, and then the tissues are sectioned. One of the keys for sectioning is the freezing speed. If the freezing speed is too slow, it is easy to produce larger ice crystal particles, thus squeezing cells or increasing the intercellular spaces, or cutting off the microstructures through the edges of the ice crystals. When the ice crystals melt, a large number of vacuoles and empty nuclei will be left, and the tissue morphology will also appear phenomena such as gap increase and structural change.

The existing methods mainly reduce the damage of the ice crystals to the cell tissues in frozen sections through the use of the cryoprotectants and the improvement of sectioning techniques (such as liquid nitrogen immersion), but can only reduce the formation of the ice crystals to a certain extent, but cannot completely inhibit the formation of the ice crystals. In addition, the existing cryosectioning methods perform dehydration by a substance such as sucrose, or perform cryoprotection by using a cryoprotectant capable of penetrating the cell membranes. These methods can only inhibit the formation of the ice crystals at low temperature, and have extremely high requirements for temperature control, otherwise they will face damage to the sample caused by crystallization or recrystallization. Moreover, the ice crystals formed during freezing will cause damage to the sample during sectioning, resulting in changes in the microstructures.

### 5. Expansion microscopy imaging

Expansion microscopy imaging is a new type of super-resolution imaging technology, which uniformly expands the biological samples with the help of hydrogel expansion, and achieves super-resolution imaging under conventional optical imaging conditions. Expansion imaging is suitable for various types of samples such as cells and tissue sections. Biological macromolecules such as proteins, nucleic acids, and lipids can be used for super-resolution imaging with the help of expansion imaging. In addition, expansion imaging is used in multi-scale combination with confocal microscope, light sheet microscope and super-resolution microscope. Under super-resolution microscope, expansion combined with sectioning can further increase the resolution to the nanometer level.

However, after expansion, the water content of the sample is extremely high, the sample is brittle and fragile, and it is difficult to carry out the next tissue processing (such as transferring, cryosectioning, etc). Moreover, the dilution of fluorescence intensity caused by expansion and the destruction of enzyme digestion process to the fluorescent proteins will lead to the weakening of fluorescence intensity, thus affecting the imaging quality. Furthermore, the existing tissue expansion methods face the problems in that it is difficult to preserve the tissues, the cryosectioning cannot be performed, and the imaging can only be performed by a low-magnification objective lens having a long working distance.

Therefore, there is an urgent need to provide a biological tissue treatment method capable of solving the above problems.

### Summary

In order to solve the above problems in the prior art, the present invention provides a hydrophilic ionic liquid formulation, which can be used in various tissue processing techniques in histology, including but not limited to a tissue clearing method, a cryopreservation method of a tissue by vitrification at ultra-low temperature, a low-temperature fluorescence enhancement method, an ice-free cryosectioning method, a cryosectioning method for an expanded tissue, and a super-resolution imaging method.

A first aspect of the present invention provides a hydrophilic amorphous ionic liquid formulation comprising the following components:
a) an ionic liquid containing a cation and an anion, the cation comprising an amino-substituted nitrogen-containing heterocyclic compound or an imine compound, and the anion comprising a compound containing carboxyl group or sulfonic group;
optionally b) an auxiliary agent for adjusting properties of component a), including one or more of antipyrine, nicotinamide, nicotinic acid, 1,4-diazabicyclo[2.2.2]octane, and a reducing agent (e.g., pyrosulfurous acid, pyrosulfite, and m-xylylenediamine, and the like); and
optionally c) water.

Herein, the reducing agent in the component b) serves to protect the other components from oxygen in the air, is preferably stably present in the ionic liquid formulation, and preferably contains potassium pyrosulfite.

Preferably, the molar ratio of said cation to said anion in component a) is close to or equal to the reciprocal of the charge ratio thereof, such that the cation and the anion are sufficiently ionized and have similar or substantially identical charge. The cation preferably comprises 1-(3-aminopropyl)-imidazole. The anion preferably comprises phthalic acid.

As described herein, the charges of a cation and an anion being "similar or substantially identical", means that the difference in the total charges of the cation and the anion is 5% or less, 2% or less, or 1% or less of the total charge of the cation. Similarly, as described herein, the molar ratio of the cation to the anion being "close to" the reciprocal of their charge ratio, refers to such that the charges of the cation and the anion are "similar or substantially identical".

In one embodiment, the amino-substituted nitrogen-containing heterocyclic compound as a cation may be a nitrogen-containing heterocyclic ring substituted with one or more aminoalkyl groups. In one embodiment, the one or more aminoalkyl groups may each independently be aminoC₁₋₈alkyl, aminoC₁₋₆alkyl, aminoC₁₋₃alkyl, aminoC₃₋₆cycloalkyl, and the like. In one embodiment, the nitrogen-containing heterocyclic ring may be a monocyclic ring or a fused ring, for example, may be a five-membered or six-membered nitrogen-containing heterocyclic ring, such as imidazole, imidazoline, imidazolidine, pyrrole, pyrroline, pyrrolidine, pyrazole, pyrazoline, pyrazolidine, triazole, tetrazole, pyridine, piperidine, piperazine, pyridazine, pyrimidine, pyrazine, triazine, and the like; and may also be eight-membered to fourteen-membered nitrogen-containing heterocyclic fused rings, such as dihydropyrrolopyrrole, tetrahydrocyclopentadienopyrrole, indole, isoindole, indoline, indazole, benzimidazole, azaindole, quinoline, isoquinoline, and hydrogenated products thereof, and the like. In one embodiment, the nitrogen-containing heterocyclic ring may also optionally contain other heteroatoms such as O and S. In one embodiment, the substitution position of the aminoalkyl group on the nitrogen-containing heterocyclic ring is not limited, but is preferably a nitrogen atom. In a preferred embodiment, the amino-substituted nitrogen-containing heterocyclic compound is an N-(amino-C₁₋₆alkyl)-five-membered or six-membered nitrogen-containing heterocyclic monocyclic ring, preferably N-(aminoC₁₋₆alkyl)-imidazole, such as 1-(3-aminopropyl)-imidazole.

In one embodiment, the compound containing carboxyl group or sulfonic group as an anion may be an aliphatic or aromatic monocarboxylic acid, dicarboxylic acid, tricarboxylic acid, monosulfonic acid, disulfonic acid, or the like. In one embodiment, the compound containing carboxyl group or sulfonic group may be carbonic acid, acetic acid, succinic acid, citric acid, benzoic acid, phthalic acid, terephthalic acid, isophthalic acid, sulfonic acid, methane-sulfonic acid, ethane-sulfonic acid, benzene sulfonic acid, benzene disulfonic acid, or the like. In a preferred embodiment, the compound containing carboxyl group or sulfonic group is benzoic acid or benzene dicarboxylic acid, for example, phthalic acid.

Preferably, the auxiliary agent of component b) can be stably present in the ionic liquid system and have a protective effect on fluorescence and tissue structure, and/or can increase the refractive index, and/or can reduce the viscosity, and/or can act an antioxidant effect, and/or can adjust the pH.

Preferably, the water of component c) is ultrapure water, preferably deoxygenated ultrapure water, for example ultrapure water subjected to deoxygenation treatment by boiling.

Preferably, based on the total weight of the ionic liquid formulation, the mass-to-mass ratio concentration of component a) is from 30 w/w% to 100 w/w%, the concentration of component b) is from 0 w/w% to 70 w/w%, the concentration of component c) is from 0 w/w% to 60 w/w%. More preferably, based on the total weight of the ionic liquid formulation, the concentration of component a) is from 50 w/w% to 70 w/w%, the concentration of component b) is from 10 w/w% to 40 w/w%, and the concentration of component c) is from 5 w/w% to 60 w/w%. Further preferably, based on the total weight of the ionic liquid formulation, the ionic liquid formulation comprises water at a concentration of 10 w/w% to 40 w/w%, 1-(3-aminopropyl)-imidazole at a concentration of 20 w/w% to 30 w/w%, phthalic acid at a concentration of 20 w/w% to 30 w/w%, nicotinamide at a concentration of 5 w/w% to 20 w/w/%, antipyrine at a concentration of 5 w/w% to 20 w/w/%, m-xylylenediamine at a concentration of 2 w/w% to 5 w/w%, and potassium pyrosulfite at a concentration of 0.2 w/w% to 1 w/w%.

Preferably, based on the total volume of the ionic liquid formulation, the mass-volume ratio concentration (w/v%) of component a) is from 35% to 95%, the mass-volume ratio concentration of component b) is from 4% to 50% (wherein the mass-volume ratio concentration of the reducing agent is preferably 0.1% to 2.0%), and the mass-volume ratio concentration of component c) is from 5% to 45%. More preferably, based on the total volume of the ionic liquid formulation, the mass-volume ratio concentration of component a) is from 75% to 90%, preferably 40% to 70%, the mass-volume ratio concentration of component b) is from 6% to 23%, preferably 25% to 40% (wherein the mass-volume ratio concentration of the reducing agent is preferably from 0.1% to 1.0%), and the mass-volume ratio concentration of component c) is from 10% to 40%, preferably 10% to 50%.

**In** a more preferred embodiment of the present invention, the ionic liquid formulation includes water having a mass-volume ratio concentration (w/v%) of 10% to 40%, 1-(3-aminopropyl)-imidazole having a mass-volume ratio concentration of 40% to 60%, potassium pyrosulfite having a mass-volume ratio concentration of 0.2% to 0.8%, phthalic acid having a mass-volume ratio concentration of 35% to 65%, antipyrine having a mass-volume ratio concentration of 8% or less (for example, 1% to 8%, 2% to 6%), and nicotinamide having a mass-volume ratio concentration of 5% to 15%, wherein the concentrations of 1-(3-aminopropyl)-imidazole as the cation and phthalic acid as the anion satisfy the molar ratio of the cation to the anion as defined in the component a).

Most preferably, in an exemplary embodiment of the present invention, the ionic liquid formulation comprises water at a mass-volume ratio concentration (w/v%) of 26%, 1-(3-aminopropyl)-imidazole at a mass-volume ratio concentration of 56%, potassium pyrosulfite at a mass-volume ratio concentration of 0.5%, phthalic acid at a mass-volume ratio concentration of 40%, antipyrine at a mass-volume ratio concentration of 5%, and nicotinamide at a mass-volume ratio concentration of 10%.

The ionic liquid formulation is in the amorphous state, and preferably remains the liquid state at room temperature.

Preferably, the refractive index of the ionic liquid formulation is 1.50 or more, preferably 1.50 to 1.55, and more preferably 1.51 to 1.54. The refractive index of the ionic liquid formulation can be achieved by adjusting the ratio of components a), b), c).

A second aspect of the present invention provides a method for preparing the ionic liquid formulation as described above, and the method comprises: formulating the components a)-c) into a formulation in a container, filling the container with an inert gas such as nitrogen, argon, helium for inert protection, and sealing the container.

**In** the above formulation and method, the sources of the pharmaceutical products and reagents used are the reagents or pharmaceutical products of analytical grade or above that are directly purchased or prepared. The ionic liquid is prepared by a method well-known to those skilled in the art, such as, but not limited to, a method for acceleration by using physical means such as ultrasound and microwave.

Alternatively, in addition to determining the ratio in advance during formulating, the formulation can reduce the water content and increase the refractive index or meet other needs by methods of freeze-drying, vacuum-drying, and adding a desiccant.

A third aspect of the present invention provides a biological tissue treatment method. **In** particular, a method for a biological tissue clearing is provided, and the method comprises: treating a delipidated biological tissue or a biological tissue section with the ionic liquid formulation described above for refractive index matching, wherein the treating preferably comprises perfusion, immersion, or infiltration. A method for the cryopreservation of a tissue by vitrification is provided, and the method comprises: infiltrating a biological tissue by using the ionic liquid formulation described above, and preserving the infiltrated biological tissue at a temperature of room temperature or lower, for example, at a temperature of 0 °C or lower, -20 °C or lower, -40 °C or lower, or -80 °C or lower. A method for low-temperature fluorescence optical enhancement imaging is provided, and the method comprises: treating a fluorescent biological tissue with the ionic liquid formulation described above, and imaging the treated biological tissue at a temperature of room temperature or lower, for example, at a temperature of 0 °C or lower, -20 °C or lower, -40 °C or lower, or -80 °C or lower. A method for an ice-free cryosectioning is provided, and the method comprises: treating a biological tissue by using the ionic liquid formulation described above, freezing the treated biological tissue, and sectioning. A method for preservation and cryosectioning of an expanded tissue is provided, and the method comprises: fixing a biological tissue with paraformaldehyde, and then expanding and secondary fixing; immersing the fixed expanded biological tissue sample in the ionic liquid formulation described above; freezing after a shape of the biological tissue sample is stabilized; and preserving or cryosectioning.

A fourth aspect of the present invention provides use of the ionic liquid formulation described above in biological tissue treatment, and the biological tissue treatment comprises one or more of tissue clearing, cryopreservation of a tissue by vitrification, low-temperature fluorescence enhancement, ice-free cryosectioning, cryosectioning of an expanded tissue, and super-resolution imaging.

### Brief Description of the Drawings

In order to more clearly explain the details and exemplary embodiments of the present invention, the embodiments of the present invention will be described in detail below with reference to the accompanying drawings, but the present invention is not limited thereto. In the drawings:
FIG. 1 is a rheometer analysis diagram of an ionic liquid of an exemplary embodiment of the present invention, showing the mechanical properties of the ionic liquid.
FIG. 2 shows a state in which an ionic liquid of an exemplary embodiment of the present invention is taken and broken after storage at minus 80 °C, demonstrating the vitrification characteristics of the ionic liquid.
FIG. 3 is a photograph of various biological tissue samples after clearing with an ionic liquid of an exemplary embodiment of the present invention.
FIG. 4 is an ex vivo brain light sheet imaging diagram after clearing with an ionic liquid of an exemplary embodiment of the present invention.
FIG. 5 is a diagram showing the clearing of sections after clearing with an ionic liquid of an exemplary embodiment of the present invention.
FIG. 6 is a spinning-disk confocal photography of tissue sections after clearing with an ionic liquid of an exemplary embodiment of the present invention, showing reduction in scattering in the XY axis and no light attenuation in the Z axis direction after clearing.
FIG. 7 shows a state at minus 80 °C of a tissue infiltrated with an ionic liquid of an exemplary embodiment of the present invention.
FIG. 8 shows fluorescence intensity data of GFPuv at different temperatures in an ionic liquid of an exemplary embodiment of the present invention.
FIG. 9 is a statistical graph of fluorescence attenuation of GFPuv at low temperature in an ionic liquid of an exemplary embodiment of the present invention.
FIG. 10 shows a flowchart of a tissue processing and sectioning process performed with an ionic liquid in Example 1 of the present application.
FIG. 11 shows a graph of the results of patch tissue processing performed with an ionic liquid in Example 2 of the present application.
FIG. 12 shows a graph of the results of the treatment of expanded clearing tissue combined with an ionic liquid in Example 3 of the present application.

### Detailed Description

Specific embodiments of the present invention are described in detail below.

One embodiment of the present invention provides a hydrophilic amorphous ionic liquid formulation comprising the following components:
a) an ionic liquid containing a cation and an anion, the cation comprising an amino-substituted nitrogen-containing heterocyclic compound or an imine compound, and the anion comprising a compound containing carboxyl group or sulfonic group;
optionally b) an auxiliary agent for adjusting properties of component a), including one or more of antipyrine, nicotinamide, nicotinic acid, and 1,4-diazabicyclo[2.2.2]octane, and a reducing agent (e.g., pyrosulfurous acid, pyrosulfite, and m-xylylenediamine, and the like); and
optionally c) water.

Herein, the reducing agent in the component b) serves to protect the other components from oxygen in the air, is preferably stably present in the ionic liquid formulation, and preferably contains potassium pyrosulfite.

The hydrophilic ionic liquid formulation of the present invention can be adjusted to have a refractive index close to the refractive index of the tissue itself and have an amorphous state and a state of high elastic modulus. No solid crystal will be formed, and the tissue can be in the vitrification state at ultra-low temperature. The hydrophilic ionic liquid formulation of the present invention can increase the quantum yield of fluorescent molecules at low temperature. The hydrophilic ionic liquid formulation of the present invention, after immersing an expanded tissue sample, is capable of displacing the moisture from the expanded tissue sample, and maintaining its expanded state.

In an exemplary embodiment of the present invention, the ionic liquid formulation comprises water at a mass-volume ratio concentration (w/v%) of 26%, 1-(3-aminopropyl)-imidazole at a mass-volume ratio concentration of 56%, potassium pyrosulfite at a mass-volume ratio concentration of 0.5%, phthalic acid at a mass-volume ratio concentration of 40%, antipyrine at a mass-volume ratio concentration of 5%, and nicotinamide at a mass-volume ratio concentration of 10%.

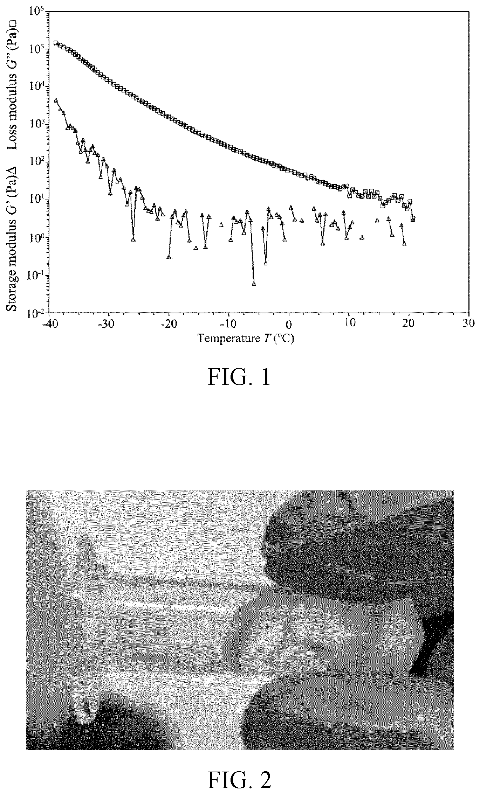

FIG. 1 is a rheometer analysis diagram of an ionic liquid formulation of the exemplary embodiment described above of the present invention, showing changes in storage modulus and loss modulus of the ionic liquid as a function of the temperature. It is demonstrated that the ionic liquid formulation of the present invention has a high elastic modulus at low temperature. The ratio value of loss modulus to storage modulus characterizes the viscosity of the ionic liquid. In the viscous flow state at higher temperature, the elevated temperature reduces the viscosity of the ionic liquid, which makes it easy to perfuse. In a state at the lower temperature, the storage modulus rises rapidly, and the material is close to the solid state. However, because its loss modulus is also higher, the material is close to the state of the semi-solid high elastic gel of the biological tissue, and is ice-free, which can be used for cryosectioning.

FIG. 2 shows a state in which an ionic liquid of the exemplary embodiment described above of the present invention is taken and broken after storage at minus 80 °C for one hour, demonstrating the vitrification characteristics of the ionic liquid.

Based on the above characteristics, the hydrophilic ionic liquid formulation of the present invention can be used in various tissue treatment methods and exhibits excellent properties.

### I. Tissue Clearing

The hydrophilic ionic liquid formulation of the present invention can be used as a refractive index matching agent in a non-deformation tissue clearing method. Specifically, the hydrophilic ionic liquid formulation of the present invention has a refractive index close to the refractive index of the tissue itself, can quickly and efficiently carry out clearing treatment on the tissue, and can avoid the problem of tissue deformation in traditional aqueous clearing and organic solvent clearing.

**In** the tissue clearing method, for the biological tissue subjected to delipidating and decolorizing treatment, the refractive index matching can be performed by adjusting the components of the formulation according to the refractive index of the main ingredients of the tissue. Moreover, the auxiliary agent component in the ionic liquid can help the ionic liquid to achieve the purpose of the clearing without deformation. The refractive index matching method can be applied to perfusion clearing, large ex vivo tissue clearing, or section clearing, and keep the macroscopic size and microscopic morphology of the tissue unchanged.

"Perfusion clearing" refers to an undiluted or diluted ionic liquid makes the refractive index matching agent sufficiently diffuse throughout the tissue using a circulation system by the way of the perfusion, to uniform the refractive index of the tissue.

"Clearing of large ex vivo tissues" refers to uniforming the refractive index of the target tissue by using an undiluted or diluted ionic liquid by the way of the immersion/infiltration.

Alternatively, for the tissue infiltrated with the diluted ionic liquid, the water content in the tissue can be reduced by the methods of freeze-drying, vacuum-drying, and adding a desiccant, to increase the refractive index of the tissue or meet other requirements.

"Section clearing" refers to refractive index matching of the tissue sections subjected to cryosectioning, paraffin sectioning, or vibration sectioning after they are delipidated. Among these, the delipidating can be performed by using methods well known to those skilled in the art.

Preferably, the tissue sections can be collected onto a glass slide and subjected to the delipidating and refractive index matching described above, based on their non-deformable properties. This operation is referred to as attaching clearing.

Attaching clearing can be carried out by the following steps:
Step 1. Prepare tissue sections by the way such as cryosectioning, paraffin sectioning, or vibration sectioning.
Step 2. Attach the tissue sections to the adhesive glass slide.
Step 3. Immerse the glass slide with the tissue sections in a delipidating reagent, wherein an aqueous delipidating reagent may be well-known to those skilled in the art.
Step 4. After completely delipidate the tissue in the tissue sections, immerse the glass slide and the tissues with a phosphate buffer to wash away the delipidating reagent. This step can be performed for three times of 20 minutes each until no foam is generated.
Step 5. Take out the glass slide and immerse it in an ionic liquid formulation diluted with water to wash away the phosphate buffer.
Step 6. Take out the glass slide, use a dust-free paper to absorb excess water, and dry the tissue sections in the air until they are slightly clearing.
Step 7. Drop the ionic liquid formulation onto the glass slide, and sufficiently infiltrate the tissue sections to make the refractive index uniform.
Step 8. Use a cover glass to seal the sections, and coat a resin to the edge of the cover glass to seal the interior. When the tissue section is thicker, a support can be used to support it.

Alternatively, as an altemative, the tissue sections can be collected into a culture dish or well plate, added with an ionic liquid for refractive index matching, and then subjected to the next attaching operation. This operation is called floating clearing.

The floating clearing described above can be carried out by the following steps:
Step 1: Prepare tissue sections by the way such as cryosectioning, paraffin sectioning, or vibration sectioning.
Step 2: Store the tissue sections in a phosphate buffer.
Step 3: Transfer the tissue sections into a delipidating reagent for delipidating.
Step 4: Transfer the tissue sections to a phosphate buffer for rinsing.
Step 5: Transfer the rinsed tissue sections to a diluted ionic liquid formulation for rinsing.
Step 6: Perform the refractive index matching which can be carried out by one of both step 6.1 and step 6.2.
Step 6.1: Transfer the tissue sections rinsed in the diluted ionic liquid to an undiluted ionic liquid and immerse therein, after the refractive index of the tissue sections is uniform, transfer the tissue sections onto the adhesive glass slide, drop the ionic liquid for sealing sections operation, and coat a resin on the edge of the cover glass to seal the interior.
Step 6.2: Transfer the tissue sections rinsed in the diluted ionic liquid to the adhesive glass slide, use a dust-free paper to absorb excess water, and dry the tissue sections in the air until they are slightly transparent. An ionic liquid is added dropwise to the glass slide, and after the refractive index of the tissue sections is uniform, a sealing sections operation is carried out, and a resin is coated on the edge of the cover slide to seal the interior.

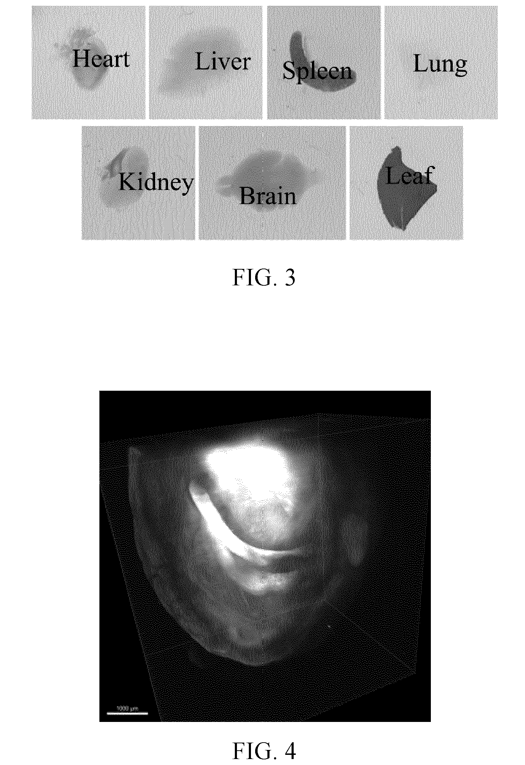

FIG. 3 is a photograph of various biological tissue samples after clearing with an ionic liquid of the exemplary embodiment described above of the present invention. Herein, the animal tissues fixed with 4% paraformaldehyde or the plant tissues without any treatment are immersed with an ionic liquid by direct infiltration. It can be seen that the heart, liver, spleen, lung, kidney, brain and plant leaf tissues immersed by the ionic liquid of the present invention are in a transparent state.

FIG. 4 is an ex vivo brain light sheet imaging diagram obtained in Example 1 after clearing with an ionic liquid of an exemplary embodiment described above of the present invention. It can be seen that the clearing based on the ionic liquid method can meet the requirements of large tissue light sheet imaging, and the effect is excellent.

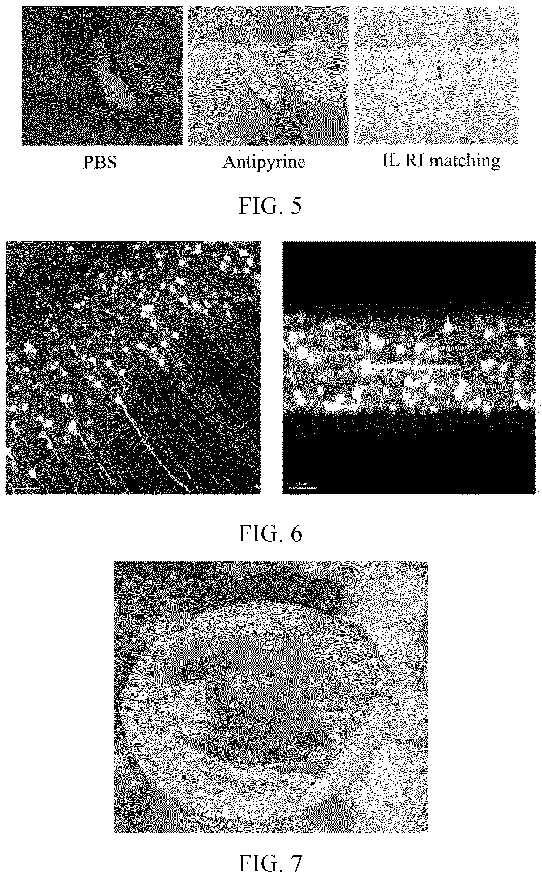

FIG. 5 is a diagram showing the clearing of a sample section after clearing with an ionic liquid of an exemplary embodiment described above of the present invention. Herein, a Zeiss Axio Scan 2 slide scanner was used to perform bright-field wide-field imaging under a 561 nm emission light filter to obtain 16-bit monochromatic brightness data. Herein, "IL RI matching" refers to the imaging result after the refractive index matching is carried out by using an ionic liquid. It can be seen that the clearing effect of the ionic liquid is very excellent.

FIG. 6 is a spinning-disk confocal photography of tissue sections after clearing with an ionic liquid of an exemplary embodiment described above of the present invention, showing reduction in scattering in the XY axis (left) and no light attenuation in the Z axis (right) direction after clearing. Herein the tissues were 200 µm tissue sections prepared by vibration sectioning of mouse brain fixed with 4% paraformaldehyde perfusion. The confocal microscope is a spinning-disk confocal microscope manufactured by Andor Company, which is photographed with a 20x lens. It can be seen that the deformation of the cleared tissue treated with the ionic liquid of the present invention on the Z axis is suppressed, and the high clearing makes the attenuation of both the excitation light and the emission light highly decreased, exhibiting an excellent imaging effect.

Therefore, the ionic liquid of the present invention can realize a non-deformation tissue clearing, and has the beneficial effects of avoiding the destruction of the tissues caused by the conventional organic solvents at high concentration and the deformation caused by it, and avoiding protein denaturation and tissue expansion caused by the conventional hydrophilic reagents as compared with the prior art. The novel ionic liquid of the present invention has a refractive index of up to 1.53, and does not cause deformation of the sample.

### II. Cryopreservation of tissue by vitrification at ultra-low temperature

The hydrophilic ionic liquid formulation of the present invention can be applied to cryopreservation of tissue by vitrification at ultra-low temperature. The hydrophilic ionic liquid formulation of the present invention does not form solid crystal regardless of temperature change due to its characteristics of amorphous morphology, and the tissue infiltrated by the ionic liquid forms a vitrification state at ultra-low temperature of minus 40 ° C or lower, and is ice-free, which does not cause damage to the microstructure of the preserved tissue. Therefore, after the tissue to be preserved is sufficiently infiltrated by the ionic liquid, it can be placed in an ultra-low temperature medical refrigerator for long-term storage, and the recrystallization formation problem caused by the rewarming of the traditional liquid nitrogen ultra-low temperature vitrified tissue is solved.

FIG. 7 shows a state at minus 80 °C of a tissue infiltrated with the ionic liquid of the exemplary embodiment described above of the present invention. Herein, the tissues are the heart, liver, spleen, lung and kidney tissues of the mouse fixed with 4% paraformaldehyde perfusion, and are placed together on the glass slides shown in the figure. After infiltration, the tissues do not form any crystals at minus 80 °C, and present a transparent glassy state. It can be seen that there is no ice crystal damage to the cleared tissue at minus 80 °C.

Therefore, the ionic liquid of the present invention can be applied to long-term cryopreservation of tissue by vitrification, and has the beneficial effects that, compared with the existing ultra-low temperature vitrification methods (such as liquid nitrogen quick freezing), the ionic liquid of the present invention has extremely low vapor pressure, does not evaporate, does not crystallize, forms a glassy state instead of crystals when the temperature is lowered, and thus does not form recrystallized ice crystals, thereby facilitating long-term cryopreservation of the samples.

### III. Low-temperature fluorescence enhancement

The hydrophilic ionic liquid formulation of the present invention can be applied to low-temperature fluorescence enhancement of the tissue samples. Since the hydrophilic ionic liquid formulation of the present invention has the amorphous characteristic described above, the sample is not damaged by the ice crystals and the microstructure is not damaged at ultra-low temperature, and thus it is possible to expose the tissues with fluorescence to the low temperature without damage. And based on the hydrophilic polarity characteristic of the ionic liquid formulation and the photochemical characteristic of increasing the quantum yield of fluorescent molecules at low temperature, the tissues with fluorescence treated or infiltrated with the ionic liquid formulation can realize fluorescence enhancement at ultra-low temperature, and can be further used for improving the optical imaging quality of photoelectric combination of cryogenic electron microscope, cryo-optical imaging for ultra-low temperature fluorescence enhancement, and for detecting weak fluorescence signals, and the like.

Alternatively, the polarity of the ionic liquid formulation at room temperature also has the property of enhancing fluorescence, and thus can also be used as a fluorescence enhancer at room temperature.

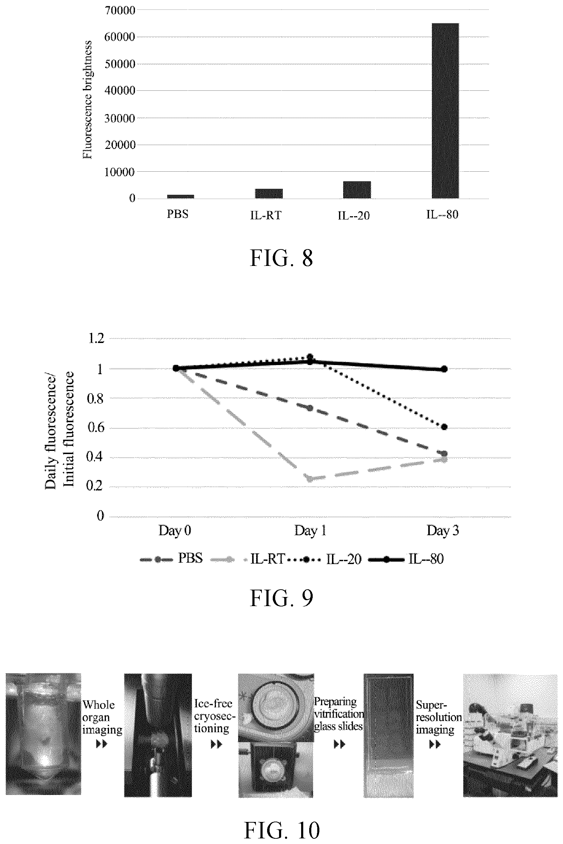

FIG. 8 shows fluorescence intensity data of GFPuv at different temperatures in the ionic liquid of the exemplary embodiment described above of the present invention, with PBS as a control. The samples to be tested were GFPuv proteins mixed with different reagents and kept overnight at the predetermined temperatures shown in the figure (room temperature, -20 °C and -80 °C) and transferred to a PerkinElmer EnVision microplate reader at the same temperature for fluorescence intensity measurement. The wavelength of the excitation light of the microplate reader is 395 nm, and the wavelength of the emission light is 506 nm. It can be seen that the fluorescence brightness of the sample at minus 80 °C (IL--80) is greatly enhanced as compared with that at room temperature (IL-RT) and minus 20 °C (IL--20).

FIG. 9 is a statistical graph showing fluorescence attenuation of GFPuv at low temperature in the ionic liquid of the exemplary embodiment described above of the present invention. FIG. 9 uses the same method as that in FIG. 8. As can be seen, by day 3, the fluorescence in the ionic liquid stored at minus 80 °C (IL--80) hardly decays, while the fluorescence in the ionic liquid stored at room temperature (IL-RT) and minus 20 °C (IL--20) shows significant decay, which proves that the ionic liquid of the present invention has excellent storage ability for fluorescent tissues at low temperature.

Therefore, the ionic liquid of the present invention has property for applying to low-temperature fluorescence enhancement. At present in the art, there is no special imaging system for fluorescence enhancement through low temperature, and most of the samples for freeze imaging are used for photoelectric combined imaging of cryogenic electron microscopy. For the samples of cryogenic electron microscope, they also face the problem of the recrystallization, and the laser that excites fluorescence may also cause the freezing and thawing of the samples, which makes this method not be popularized too much. However, the ionic liquid used in the invention does not weaken the intensity of the fluorescent protein, instead can enhance the intensity of the fluorescent protein at low temperature, and does not evaporate or crystallize, and has little damage to the sample.

### IV. Ice-free cryosectioning

The hydrophilic ionic liquid formulation of the present invention can be applied to prepare frozen sections of tissue samples without ice crystal damage. The hydrophilic ionic liquid formulation of the present invention has the characteristics of being amorphous and having a high shear elastic modulus state (see FIG. 1), and can avoid the damage to the microstructure caused by the formation of ice crystals due to the sample freezing in the frozen section. Therefore, the tissue treated or infiltrated by the ionic liquid formulation can realize the frozen section without ice crystal damage in a specific temperature range.

In contrast, the cryosectioning method of the prior art uses the substances such as sucrose for dehydration, or uses a cryoprotectant that can penetrate the cell membrane for cryoprotection, which can only inhibit the formation of ice crystals at low temperature, and has extremely high requirements for temperature control, otherwise it will result in damaging to the sample due to crystallization or recrystallization. And ice crystals will still form during freezing, resulting in damage to the sample and changes in the microstructure caused by the sectioning process. However, the ionic liquid of the present invention will form a glassy state instead of crystalline when the temperature is lowered (see FIG. 2), so that it can be used for cryosectioning without ice crystal damage.

### V. Cryosectioning and super-resolution imaging of expanded tissues

The hydrophilic ionic liquid formulation of the present invention can be applied to cryosectioning and super-resolution imaging of the expanded tissue. After the expanded biological tissue is subjected to secondary fixing by gel, it can be immersed in the ionic liquid formulation of the present invention to replace moisture and still maintain the expanded state. Using the state transformation of the ionic liquid at low temperature, the biological tissue will be transformed into an amorphous highly-elastic state, and then ultra-thin cryosectioning can be performed to achieve super-resolution imaging of an ordinary optical microscope, which combined with a confocal microscope, a light sheet microscope, an super-resolution microscope, and the like, can further increase the resolution to the nanometer level. At the same time, the low temperature makes the increase in the fluorescence intensity, which can alleviate the problem of the dilution of fluorescence brightness caused by the sample expansion.

The present invention has solved the problems such as traditional difficult preservation of the expanded tissue, too fragile sample after being expanded, difficulty in carrying out next tissue processing (such as transferring, sectioning, etc), the dilution of fluorescence intensity caused by expansion, so that the expanded tissue sample can also be adapted to a high-magnification objective lens with a short working distance through sectioning, and super-resolution imaging that breaks through the limit of an optical microscope is realized.

### Example

The following examples provide some embodiments of the present invention, but the embodiments of the present invention are not limited to the following examples.

### Example 1

The ionic liquid of the exemplary embodiment described above of the present invention was used to perform the perfusion clearing of the brain of the mouse, and super-resolution imaging of the cleared tissue sections was performed in combination with freeze drying and cryosectioning operations. The basic flowchart was shown in FIG. 10, and the specific operations were as follows.
1. Mice were subjected to transcardiac perfusion with pre-cooled heparin-saline solution until the blood was sufficiently drained.
2. The mice were subjected to transcardiac perfusion with 4% paraformaldehyde solution containing 0.01 M phosphate buffer for 40 minutes.
3. The mice were subjected to systemic delipidating perfusion with a delipidating reagent until they were completely delipidated.
4. The mice were subjected to transcardiac perfusion with an aqueous solution of 4-fold diluted ionic liquid and left overnight.
5. The brain was taken and the brain of the mouse was placed in 1.5-fold diluted ionic liquid.
6. The liquid and the brain of the mouse were frozen in a refrigerator at minus 80 °C and then freeze-dried in a vacuum freeze-dryer until the brain of the mouse was transparent.
7. The transparent brain of the mouse was imaged by using a light sheet microscope, wherein the imaging liquid was the ionic liquid itself.
8. The transparent brain of the mouse was embedded with a cryoembedding agent, and then sufficiently frozen at minus 80 °C.
9. After the freezing was completed, the brain of the mouse was transferred to a microtome cryostat with the help of dry ice.
10. The brain of the mouse was sectioned into 50 um thin sections at minus 40 °C and the sections were attached to gelatin-coated adhesive glass slides.
11. A super-resolution imaging of the sections was performed by using an Airyscan microscope to obtain data.

The imaging results are shown in FIG. 4. It can be seen that the clearing method based on the ionic liquid can meet the requirements of large tissue light sheet imaging, and the effect is excellent.

In addition, the heart, liver, spleen, lung, kidney, and plant leaves were treated by using similar methods, and their imaging results are shown in FIG. 3. It can be seen that various tissues immersed in the ionic liquid of the present invention are in a transparent state.

### Example 2

The ionic liquid of the exemplary embodiment described above of the present invention was used to perform non-deformation clearing treatment on the attached tissue sections, and the specific operations were as follows:
1. The prepared tissue sections were immersed in a delipidating reagent for 30 minutes.
2. The tissue sections were immersed in 4-fold diluted ionic liquid 3 times for 20 minutes each time to wash away the delipidating reagent.
3. The glass slides were wiped dry and the brain sections were kept in the air until they are slightly transparent.
4. An ionic liquid was added dropwise onto tissue sections for refractive index matching, which took about 1 minute.
5. After the tissue sections were transparent, the sealing sections operation was carried out.
6. After sealing sections, the tissue sections were photographed with an Andor Dragonfly spinning disk confocal microscope 60x.

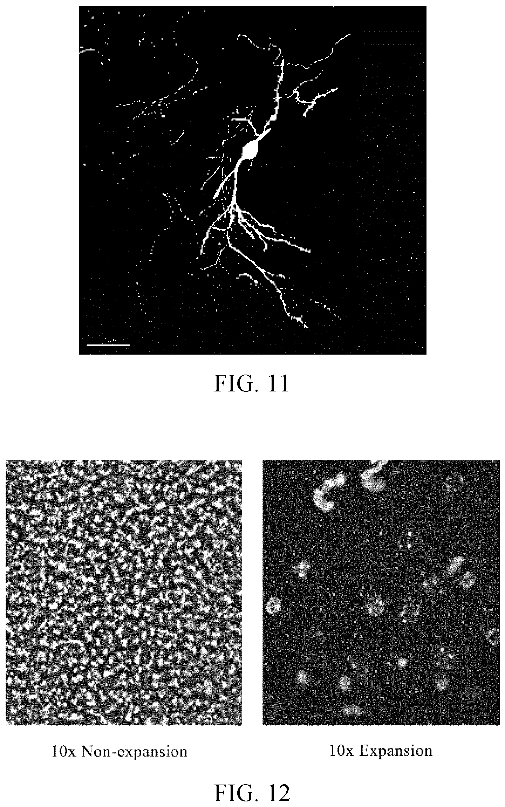

The imaging results of this example are shown in FIG. 11, in which the tissues are not deformed after they are treated with the ionic liquid.

### Example 3

The ionic liquid of the exemplary embodiment described above of the present invention was used to perform the cryosectioning of the expanded tissues, and the specific operations were as follows:
1. The mice were subjected to transcardiac perfusion with the paraformaldehyde, the brain was stripped, post-fixed for one day, and then transferred into glycine solution. The brain sections were obtained by vibration sectioning and stored at 4 °C.
2. AcX was suspended in anhydrous DMSO at a concentration of 10 mg/mL. AcX was diluted to 0.1 mg/mL with PBS before use, and the sample was incubated in the diluted AcX in a shaker at room temperature for at least 6 h.
3. Concentrated ammonium persulfate initiator and tetramethylethylenediamine were added to a monomer solution, each at most 0.2% (w/w).The inhibitor 4-hydroxy-TEMPO was added, and the tissue sections were incubated with the monomer solution plus APS/TEMED for 30 minutes at 4 °C and then transferred to a humidified incubator at 37 °C for 2 hours for gelation.
4. The gel was completely immersed in a protease solution overnight at room temperature/37 °C for 4 hours.
5. The digested gel was placed in excess double deionized water for 0.25-2 hours to expand it, which was repeated 3-5 times until the size of the expanded sample reached a steady state.
6. The expanded gel was placed in 2 mL of PEG-DA solution and protected from light at room temperature overnight. After that, the gel was removed from the PEG-DA and the excess PEG-DA solution on the surface was absorbed, and the gel was irradiated by using 4 mW/cm² Ultraviolet light for 10 min.
7. The gel was infiltrated in the ionic liquid until the shape was stable, sectioned, and imaged by using a 10x lens.

The imaging results of this example are shown in FIG. 12.

The technical idea and specific embodiments of the present invention have been described above, but it should be understood that the above specific embodiments do not limit the scope of the present invention in any way. It will be understood by those skilled in the art that various modifications and/or variations may be made to the invention shown in the Detailed Description of the Embodiments without departing from the spirit of the invention, and that the modified and/or modified embodiments are also contemplated within the scope of the invention. Accordingly, the embodiments of the present invention are merely illustrative and non-limiting.

## Claims

1. A hydrophilic amorphous ionic liquid formulation, comprising the following components:
a) an ionic liquid containing a cation and an anion, the cation comprising an amino-substituted nitrogen-containing heterocyclic compound or an imine compound, and the anion comprising a compound containing carboxyl group or sulfonic group;
optionally b) an auxiliary agent for adjusting properties of component a), comprising one or more of antipyrine, nicotinamide, nicotinic acid, 1,4-diazabicyclo[2.2.2]octane, pyrosulfurous acid, pyrosulfite, and m-xylylenediamine; and
optionally c) water.

2. The ionic liquid formulation according to claim 1, wherein a molar ratio of the cation to the anion in component a) is close to or equal to the reciprocal of a charge ratio thereof, such that the cation and the anion are sufficiently ionized and have similar or substantially identical charge.

3. The ionic liquid formulation according to claim 1 or 2, wherein the cation comprises a nitrogen-containing heterocyclic ring substituted with one or more aminoalkyl groups, wherein the one or more aminoalkyl groups are preferably each independently aminoC₁₋₈alkyl, aminoC₁₋₆alkyl, aminoC₁₋₃alkyl, or aminoC₃₋₆cycloalkyl, and the nitrogen-containing heterocyclic ring is preferably a five-membered or six-membered nitrogen-containing heterocyclic monocyclic ring or an eight-membered to fourteen-membered nitrogen-containing heterocyclic fused ring; and preferably, the cation comprises an N-(aminoC₁₋₆alkyl)-five-membered or six-membered nitrogen-containing heterocyclic monocyclic ring, for example 1-(3-aminopropyl)-imidazole.

4. The ionic liquid formulation according to claim 1 or 2, wherein the anion comprises an aliphatic or aromatic monocarboxylic acid, a dicarboxylic acid, a tricarboxylic acid, a monosulfonic acid or a disulfonic acid, and preferably comprises benzoic acid or benzenedicarboxylic acid, for example phthalic acid.

5. The ionic liquid formulation according to claim 1 or 2, wherein component c) is ultrapure water, preferably deoxygenated ultrapure water, for example, ultrapure water subjected to deoxygenation treatment by boiling.

6. The ionic liquid formulation according to claim 1 or 2, wherein the pyrosulfite is potassium pyrosulfite.

7. The ionic liquid formulation according to claim 1 or 2, wherein a concentration of component a) is from 30 w/w% to 100 w/w%, a concentration of component b) is from 0 w/w% to 70 w/w%, and a concentration of component c) is from 0 w/w% to 60 w/w%, based on the total weight of the ionic liquid formulation;
preferably, the concentration of component a) is from 50 w/w% to 70 w/w%, the concentration of component b) is from 10 w/w% to 40 w/w%, and the concentration of component c) is from 5 w/w% to 60 w/w%, based on the total weight of the ionic liquid formulation.

8. The ionic liquid formulation according to claim 1 or 2, comprising: based on the total weight of the ionic liquid formulation, water at a concentration of 10 w/w% to 40 w/w%, 1-(3-aminopropyl)-imidazole at a concentration of 20 w/w% to 30 w/w%, phthalic acid at a concentration of 20 w/w% to 30 w/w%, nicotinamide at a concentration of 5 w/w% to 20 w/w%, antipyrine at a concentration of 5 w/w% to 20 w/w%, m-xylylenediamine at a concentration of 2 w/w% to 5 w/w%, and potassium pyrosulfite at a concentration of 0.2 w/w% to 1 w/w%.

9. The ionic liquid formulation according to claim 1 or 2, wherein the refractive index of the ionic liquid formulation is 1.50 or more, preferably 1.50 to 1.55, and more preferably 1.51 to 1.54.

10. A method for preparing the ionic liquid formulation according to any one of claims 1 to 9, comprising:
formulating the components a)-c) into a formulation in a container, and
filling the container with an inert gas such as nitrogen, argon, helium for inert protection, and sealing the container.

11. A method for a biological tissue clearing, comprising:
treating a delipidated biological tissue or biological tissue section with the ionic liquid formulation according to any one of claims 1 to 9 for refractive index matching; wherein the treating preferably comprises perfusion, immersion, or infiltration.

12. A method for the cryopreservation of a tissue by vitrification, comprising:
infiltrating a biological tissue by using the ionic liquid formulation according to any one of claims 1 to 9, and preserving the infiltrated biological tissue at a temperature of room temperature or lower, for example, at a temperature of 0 °C or lower, -20 °C or lower, -40 °C or lower, or -80 °C or lower.

13. A method for low-temperature fluorescence optical enhancement imaging, comprising:
treating a biological tissue with fluorescence by using the ionic liquid formulation according to any one of claims 1 to 9, and imaging the treated biological tissue at room temperature or lower, for example, at a temperature of 0 °C or lower, -20 °C or lower, -40 °C or lower, or -80 °C or lower.

14. A method for an ice-free cryosectioning, comprising:
treating a biological tissue by using the ionic liquid formulation according to any one of claims 1 to 9, freezing the treated biological tissue, and sectioning.

15. A method for preservation and cryosectioning of an expanded tissue, comprising:
primary fixing a biological tissue, and then expanding and secondary fixing; immersing the expanded biological tissue sample after the secondary fixing in the ionic liquid formulation according to any one of claims 1 to 9; freezing the biological tissue sample after a shape of the biological tissue sample is stabilized; and preserving or cryosectioning; wherein the primary fixing is preferably carried out with a paraformaldehyde, and the secondary fixing is preferably carried out with a gel.

16. Use of the ionic liquid formulation according to any one of claims 1 to 9 in biological tissue treatment, wherein the biological tissue treatment comprises one or more of tissue clearing, cryopreservation of a tissue by vitrification, low-temperature fluorescence enhancement, ice-free cryosectioning, cryosectioning of an expanded tissue, and super-resolution imaging.
